(19) Europäisches Patentamt
European Patent Office
Office européen des brevets

(11) Veröffentlichungsnummer: **0 050 220**
**A1**

# (12) EUROPÄISCHE PATENTANMELDUNG

(21) Anmeldenummer: **81107474.9**

(22) Anmeldetag: **21.09.81**

(51) Int. Cl.³: **C 07 D 251/34**, C 08 K 5/34

(30) Priorität: **16.10.80 DE 3039059**

(43) Veröffentlichungstag der Anmeldung: **28.04.82**
**Patentblatt 82/17**

(84) Benannte Vertragsstaaten: **BE DE FR GB IT NL**

(71) Anmelder: **BASF Aktiengesellschaft,**
**Carl-Bosch-Strasse 38, D-6700 Ludwigshafen (DE)**

(72) Erfinder: **Naarmann, Herbert, Dr., Haardtblick 15,**
**D-6719 Wattenheim (DE)**
Erfinder: **Penzien, Klaus, Dr., Bensheimer Ring 18,**
**D-6710 Frankenthal (DE)**
Erfinder: **Brandstetter, Franz, Dr., Ritterbuechel 45,**
**D-6730 Neustadt (DE)**
Erfinder: **Lindenschmidt, Gerhard, Dr., Buchenweg 11,**
**D-6906 Leimen (DE)**
Erfinder: **Seiler, Erhard, Dr., Erpolzheimer Strasse 1,**
**D-6700 Ludwigshafen (DE)**

(54) **Halogen enthaltende s-Triazinverbindungen, Verfahren zur Herstellung derselben und deren Verwendung für die Flammfestausrüstung von thermoplastischen Kunststoffen.**

(57) Die Erfindung betrifft Halogen enthaltende s-Triazinverbindungen der folgenden allgemeinen Formel I

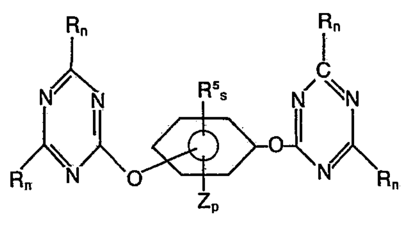

wobei die Reste und Indices folgende Bedeutung haben:

$R_n$: OH, $R^1$, $R^2$ oder $R^3$

$R^1$: 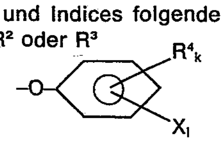

wobei gilt:

$R^4$: niedere Alkylgruppe oder niedere Halogenalkylgruppe

X: Brom oder Chlor

k, l: 0 oder ganze Zahlen von 1 bis 5 und ferner k+l max. 5;

$R^2$: 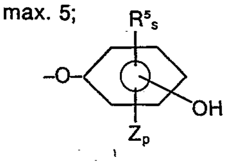

wobei gilt:

$R^5$: niedere Alkylgruppe oder niedere Halogenalkylgruppe

Z: Brom oder Chlor

s, p: 0 oder ganze Zahlen von 1 bis 4 und ferner s+p = max. 4

$R^3$: 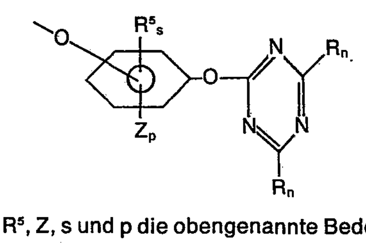

wobei $R_n$, $R^5$, Z, s und p die obengenannte Bedeutung haben und ferner gilt l+p = 1.

Die beanspruchten Verbindungen werden hergestellt durch Umsetzen von Cyanurchlorid mit einem Phenol der Formel

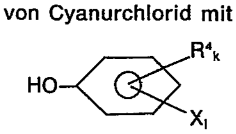

(Fortsetzung nächste Seite)



ACTORUM AG

und Benzolderivaten der Formel

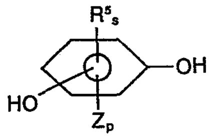

in Gegenwart einer Base.

Die dabei erhaltenen Produkte finden für die Flammfestausrüstung von thermoplastischen Kunststoffen, insbesondere von mit Kautschuk schlagfest modifizierten Styrol-Acrylnitril-Copolymerisaten (ABS-Harzen) Verwendung.

Halogen enthaltende s-Triazinverbindungen, Verfahren zur Herstellung derselben und deren Verwendung für die Flammfestausrüstung von thermoplastischen Kunststoffen

Die Erfindung betrifft Halogen enthaltende s-Triazinverbindungen mit zwei oder mehreren s-Triazinringen. Diese Verbindungen werden hergestellt durch Umsetzung von Cyanurchlorid mit einem Phenol und einem 2 OH-Gruppen enthaltenden Benzolderivat in Gegenwart einer wäßrigen Base und unter Verwendung eines polaren Lösungsmittels. Die beanspruchten Verbindungen werden für die Flammfestausrüstung von thermoplastischen Kunststoffen verwendet.

Zum Stand der Technik nennen wir:

1. FR-PS 1 566 675
2. US-PS 3 843 650
3. JA-OS 25 232/1972
4. DE-OS 27 27 650

Aus (1) sind Halogen enthaltende s-Triazinverbindungen mit einem s-Triazinring bekannt. So ist in (1) bzw. (2) oder (3) das Tris(2,4,6-tribromphenoxy)-s-triazin beschrieben und dessen Verwendung als Flammschutzmittel für Polyolefine, Polystyrole, ABS, PVC usw. aus (2) bzw. (3) bekannt. Diese Tris-(polyhalogenphenoxy)-s-triazine haben eine hohe Entflammungsverzögerungswirkung. Sie zeigen jedoch keine genügend hohe thermische Stabilität und führen zum Ausblühen beim Mischen mit synthetischem Harz. Es ist weiterhin aus (4) bekannt, daß mehrkernige Triazinverbindungen aus Cyanurchlorid, Phenolen und Bisphenolen hergestellt werden können. Das bevorzugte, leicht zugängliche und bromierbare Bisphenol-A enthält zwei Phenylringe, die über eine $-C(CH_3)_2$-Gruppe verknüpft sind. Diese zur Verknüpfung der aromatischen Reste eingeführte aliphatische, leicht brennbare Gruppe ist bei Anwendung der bekannten haloge-

Vo/BL

nierten s-Triazine auf dem Gebiet des Flammschutzes ohne Wirkung und ohne Vorteile. Es bestand daher die Aufgabe, einfachere s-Triazinsysteme enthaltende Verbindungen vorzuschlagen und ein Verfahren für die Herstellung dieser Verbindungen zu entwickeln, das ausgehend von Cyanurchlorid und halogenierten Phenolen zu s-Triazinverbindungen führt, die möglichst keine leichtbrennbaren Molekülgruppen enthalten und einen möglichst hohen Halogengehalt aufweisen.

Die Aufgabe wurde gelöst durch Auffinden von s-Triazinverbindungen der allgemeinen Formel I gemäß Patentanspruch 1, sowie durch ein Verfahren zur Herstellung dieser Verbindungen aus Cyanurchlorid, Phenolen (III) und zweifach hydroxysubstituierten Benzolderivaten (IV) unter der Einwirkung von Basen.

Die Erfindung betrifft daher Halogen enthaltende s-Triazinverbindungen gemäß Patentanspruch 1.

Die Erfindung betrifft ferner ein Verfahren zur Herstellung von Verbindungen gemäß Formel I von Patentanspruch 1 oder Mischungen von Verbindungen der allgemeinen Formel I und Halogen enthaltenden Tri(phenoxi)-s-triazinen der Formel II.

Für die Herstellung der erfindungsgemäßen Verbindungen der Formel I bzw. Mischungen von Verbindungen der Formel I mit denen der Formel II kommen Phenole der allgemeinen Formel III in Betracht.

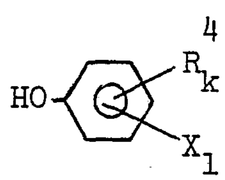

III

Phenole, die für die Umsetzung geeignet sind, sind die Halogenphenole, wie Monobromphenol, Monochlorphenol, Dibromphenol, Dichlorphenol, Tribromphenol, Trichlorphenol, Pentabromphenol, Pentachlorphenol, Kresol, Monobromkresol, Monochlorkresol, Dibromkresol, Dichlorkresol, Tribromkresol, Trichlorkresol, die Halogenalkylphenole, wie (2-Bromäthyl)dibromphenol, (2-Chloräthyl)dichlorphenol usw. Von den genannten halogenierten Phenolen wird besonders bevorzugt das Tribromphenol, das Trichlorphenol, das Pentabromphenol und das Pentachlorphenol verwendet. Auch Mischungen von zwei oder mehreren der genannten Phenole können für die Herstellung der Verbindung I oder deren Mischungen mit Verbindung II verwendet werden.

Als zweifach hydroxy-substituierte Benzolderivate der allgemeinen Formel IV kommen insbesondere Tetrabromhydrochinon, Tetrabrombrenzcatechin und Tetrabromresorcin in Frage.

Zur Herstellung der erfindungsgemäßen Halogen enthaltenden s-Triazinverbindungen werden die halogenierten Phenole der allgemeinen Formel III mit den zweifach hydroxy-substituierten Benzolderivaten der allgemeinen Formel IV und mit Cyanurchlorid in Gegenwart einer Base, bevorzugt unter Verwendung eines polaren organischen Lösungsmittels, bei Temperaturen im Bereich von 0 bis 100°C umgesetzt. Als Säurefänger werden bei dieser Umsetzung die Hydroxide der Alkali- oder Erdalkalimetalle, wie Natriumhydroxid, Kaliumhydroxid, Calciumhydroxid oder deren Carbonate verwendet. Besonders bevorzugt findet eine wäßrige Lösung von Natriumhydroxid Einsatz.

Für die Umsetzung werden bevorzugt polare organische Lösungsmittel wie Halogenkohlenwasserstoffe, insbesondere Äther, cyclische Äther, Ketone, Ester oder Alkohole ange-

wendet. Es ist besonders bevorzugt, cyclische Äther wie 1,4-Dioxan oder Tetrahydrofuran zu verwenden.

Bei der Umsetzung der Reaktanten a, b und c gemäß Patentanspruch 2 wird der Ansatz bevorzugt so ausgelegt, daß die Zahl der Hydroxyläquivalente aus dem Phenol der allgemeinen Formel III und dem zweifach OH-substituierten Benzolderivat der allgemeinen Formel IV gleich der Zahl der Chloräquivalente des Cyanurchlorids ist. Bei Verwendung von 1 Mol Cyanurchlorid werden dementsprechend 3 Mol Alkalilauge benötigt. In einer bevorzugten Ausführungsform zur Herstellung der erfindungsgemäßen halogenhaltigen s-Triazinverbindungen werden die Reaktanten Cyanurchlorid, Phenol der Formel III und das 2 OH-Gruppen enthaltende Benzolderivat der allgemeinen Formel IV in Tetrahydrofuran vorgelegt. Danach wird die benötigte und berechnete Menge wäßriger Natronlauge bei Temperaturen im Bereich zwischen 20 und 50°C zugegeben. Einige Stunden Nachrührzeit unter Verwendung einer Rückflußkühlung vervollständigen die Umsetzung. Bei der Aufarbeitung des Reaktionsansatzes ist es wesentlich, daß das entstandene Natriumchlorid entfernt wird. Dies gelingt auf einfache Weise durch Einrühren der Reaktionsmischung in einen Überschuß an vorgelegtem Wasser oder eines Methanol-Wasser-Gemisches. Anschließend wird das halogenierte s-Triazin durch Filtration isoliert und getrocknet. Eine Variante zur Aufarbeitung des Reaktionsproduktes besteht darin, daß nach der Reaktion das mit Wasser mischbare Lösungsmittel abdestilliert wird und der Rückstand mit einem chlorierten Kohlenwasserstoff als Lösungsmittel aufgenommen wird. Die dabei entstehende Suspension wird mit Wasser gewaschen, die wäßrige und die organische Phase werden voneinander getrennt und die organische Phase wird eingedampft. Dabei fallen die halogenierten s-Triazine als verhältnismäßig niedrigviskose, farblose bis gelbe Schmelzen an. Eine weitere Variante zur

Herstellung der erfindungsgemäßen Verbindungen ist die Verwendung von Mischungen aus chlorierten Kohlenwasserstoffen und Wasser als Reaktionsmedium, wobei ein Zusatz von Phasentransferkatalysatoren zweckmäßig ist. Die Reihenfolge der Zugabe der Reaktanten ist insofern kritisch, als das Chlor des Cyanurchlorids in Wasser, insbesondere in dem alkalischen Milieu, durch OH-Gruppen substituiert wird. Maßnahmen zur Zurückdrängung dieser weniger erwünschten Nebenreaktion, die zu halogenierten s-Triazinen mit erhöhter OH-Zahl führt, bestehen darin, daß man entweder das Cyanurchlorid erst nach Vorlage der übrigen Reaktionspartner zugibt oder mit der Zugabe des Alkalis erst nach Vorlage aller anderen Reaktionspartner beginnt. Außerdem soll die Anwendung höherer Reaktionstemperaturen, insbesondere zu Beginn der Umsetzung, vermieden werden.

Maßgeblich für die Eigenschaften der erfindungsgemäßen s-Triazinderivate ist das bei ihrer Herstellung angewendete Molverhältnis von Cyanurchlorid zu zweifach hydroxysubstituiertem Benzolderivat der allgemeinen Formel IV. Das Molverhältnis von Cyanurchlorid a) zu Benzolderivat c) soll 1 bis 20, vorzugsweise 2 bis 8 betragen. Bei Anwendung eines niedrigen Molverhältnisses von 1 bis 2 sind die Reaktionsprodukte vernetzt oder hochschmelzend, bei einem Molverhältnis größer als 8 bis zu 20 enthalten die Reaktionsprodukte im wesentlichen die Tris(phenoxy)-triazinverbindung der allgemeinen Formel II. Bei Anwendung von Molverhältnissen im Bereich von größer als 2 bis zu 8 bestehen die Reaktionsprodukte aufgrund von Molgewichtsbestimmungen im wesentlichen aus einer Mischung der s-Triazinverbindung der Formel II und einer halogenhaltigen s-Triazinverbindung mit 2 Triazinkernen der allgemeinen Formel I.

Die erfindungsgemäßen Stoffe bzw. Stoffmischungen werden bevorzugt für die Flammfestausrüstung von thermoplastischen Kunststoffen angewendet. Dabei hat sich herausgestellt, daß bei genügendem Halogen - insbesondere Bromgehalt des s-Triazins und des Kunststoffs ein ausreichender Flammschutz nach den Vorschriften der Underwriter Laboratories zwecks Einordnung in eine der Brandklassen 94 VE-0, 94 VE-1 oder 94 VE-2, geprüft im vertikalen Brandtest, erreicht wird. Für die Flammfestausrüstung werden vorzugsweise die üblichen Synergisten wie $Sb_2O_3$, $SnO_2$, $Fe_2O_3$ u.dgl. in dem Fachmann bekannten Mengen zusammen mit dem Brandschutzmittel angewendet.

Als thermoplastische Kunststoffe für die Flammfestausrüstung kommen in Betracht Polyäthylen, Polypropylen, Polyisobutylen, Polystyrol sowie die Copolymerisate des Styrols mit Acrylnitril, ferner Polyamide und die Polyester. Besonders bevorzugt werden schlagfeste Thermoplaste wie schlagfestes Polystyrol, sowie die mit Kautschuken schlagfest modifizierten Styrol-Acrylnitril-Copolymerisate (ABS-Harze) und von den Polyestern das Polybutylenterephthalat. Die Flammschutzmittel werden dabei in Mengen von 2 bis 25 Gewichtsteilen, bezogen auf 100 Teile des thermoplastischen Kunststoffes, angewendet. Die Synergisten sind in Mengen von 1 bis 10 Gewichtsteilen, bezogen auf 100 Gewichtsteile thermoplastischen Kunststoff, in der Regel wirksam.

Die flammfest auszurüstenden thermoplastischen Kunststoffe können außer dem Flammschutzmittel und Synergisten übliche Zusatzstoffe wie Stabilisatoren sowie Füllstoffe, Farbpigmente, Gleitmittel, Weichmacher, Antistatika oder Treibmittel in üblichen, dem Fachmann bekannten Mengen enthalten. Die Einarbeitung des Flammschutzmittels, des Synergisten sowie der eventuell verwendeten Zusatzstoffe

kann nach geeigneten und bekannten Mischverfahren, z.B. in Extrudern, Knetern oder Walzen, erfolgen. Insbesondere können auch Konzentrate des Flammschutzmittels, des Synergisten in dem gewünschten thermoplastischen Kunststoff hergestellt werden.

Es wurde gefunden, daß Flammschutzmittelmischungen mit einem Gehalt von mehr als 53 Gew.% an Tris(tribromphenoxy)-s-triazin der allgemeinen Formel II Migrationsprobleme bei ABS-Polymerisaten verursachen.

Die Prozentgehalte an Tris(tribromphenoxy)-s-triazin (Formel II) im bromierten s-Triazingemisch lassen sich unter bestimmten Annahmen nach folgender Gleichung berechnen:

$$\text{\%-Gehalt der Formel II} = \frac{(M-2) \cdot 100 \cdot 1068}{(M-2) \cdot 1068 + 1900}$$

Hierbei bedeutet M das Molverhältnis (a:c) von Cyanurchlorid zu dem insbesondere verwendeten Tetrabromhydrochinon als Repräsentant der allgemeinen Formel IV. Nach Molgewichtsbestimmungen des Reaktionsgemisches gilt diese Gleichung mit guter Näherung für $M \geqq 2$, wobei Tribromphenolnatrium als weiterer Reaktionspartner diente.

Die beanspruchten Mengenbereiche sind in diesem Sinne auch als ungefähr zu betrachten.

Bei der Flammfestausrüstung von ABS-Harzen läßt sich eine besonders gute Mechanik der Formmasse bei gleichzeitiger Migrationsfreiheit dann erzielen, wenn die erfindungsgemäßen halogenhaltigen s-Triazinverbindungen der allgemeinen Formeln I und II in einer Mischung angewendet werden, die 22 bis 53 Gew.% an Verbindung der allgemeinen Formel II, bezogen auf die Summe von der Verbindung I und II,

aufweist; die %-Anteile II wurden nach der Näherungsformel im letzten Absatz für M = a/c = 2,5 bis M = a/c = 4 errechnet.

Für die Flammfestausrüstung mit den erfindungsgemäßen s-Triazinverbindungen wurde ein ABS verwendet, das 24 Gewichtsteile Polybutadien und eine Matrix mit einer Viskositätszahl von 55 bzw. 80 enthielt. Die Bestimmung der Viskositätszahl erfolgte analog DIN 53 726; dabei wurden 0,5 g Material in 100 ml Dimethylformamid gelöst. Der Styrolanteil in der Matrix lag bei 65 Gew.-%, der Acrylnitrilanteil bei 35 Gew.-%.

Die thermoplastischen ABS-Polymerisate wurden mit jeweils 5 Gewichtsteilen eines Synergisten (Antimontrioxid) und 20 Gewichtsteilen eines Flammschutzmittels gemäß der vorliegenden Erfindung auf einem Zweiwellenextruder gemischt.

Zur Prüfung der mechanischen Eigenschaften des ABS, das die erfindungsgemäßen Flammschutzmittel enthält, wurden auf einer Spritzgußmaschine vom Typ Arberg Allrounder 200 bei 250°C Rundscheiben von 2 mm Dicke hergestellt. Die Schädigungsarbeit wurde nach DIN 53 443, Blatt 1, ermittelt.

Zur Prüfung der Oberflächenbelagsbildung an Formteilen aus den erfindungsgemäßen Formmassen und an solchen aus Vergleichsversuchen wurden bei 230°C Preßplatten mit den Abmessungen 100 x 100 x 2 mm hergestellt. Die Aufheizzeit betrug 3 Minuten, die Preßzeit bei 230°C 8 Minuten. Die Preßplatten wurden bei 60°C in einem Trockenschrank gelagert. Nach bestimmten Zeitabschnitten wurde visuell kontrolliert, ob sich ein Oberflächenbelag gebildet hatte oder nicht.

Polybutylenterephthalat mit einer relativen Viskosität $\eta_{rel}$ von 1,67 (gemessen 0,5 %ig in Phenol/o-Dichlorbenzol 60:40 bei 25°C) wurde zusammen mit 10 Gew.-%, bezogen auf Polymer der bromierten Triazinverbindungen, die nicht mehr als 70 Gew.-% an Tris(tribromphenoxi)-s-triazin enthalten, sowie 6 Gew.-%, bezogen auf Polymer an Antimontrioxid als Flammschutzsynergist, in einem Zweiwellenextruder aufgeschmolzen. In die Schmelze wurden 30 Gew.-% Glasfasern, bezogen auf die Gesamtmischung, eingearbeitet. Die homogene Mischung wurde ausgetragen, abgekühlt und granuliert. Aus dem Granulat wurden Flachstäbe von 1/8 und 1/16" Dicke gespritzt und nach UL 94 geprüft. Das Produkt ist in der Brandklasse V-O nach UL 94 einzuordnen. Je 2 Prüfstäbe wurden 1, 3 und 7 Tage bei 170°C im Umlufttrockenschrank gelagert. Es wird eine leichte Farbvertiefung von hellelfenbein nach hellbeige festgestellt. Ein Belag auf der Oberfläche ist nicht festzustellen. Die Produkte zeigen bei Prüfung nach der FTZ-Vornorm 547 PV 1 vom Juni 71 keine Kontaktschädigung im Niederspannungsbereich.

Die Erfindung wird nachstehend anhand von Beispielen und Vergleichsversuchen näher erläutert. Die in den Beispielen und Versuchen genannten Teile und Prozente beziehen sich, sofern nichts anderes vermerkt ist, auf das Gewicht (vgl. Tabelle 2, in der auch die Ergebnisse der Vergleichsversuche A bis D enthalten sind.

Beispiele 1 - 8

In einem Glaskolben werden 0,2 Mol Cyanurchlorid, x Mol Tetrabromhydrochinon und y Mol Tribromphenol in 400 ml Tetrahydrofuran vorgelegt. Dann läßt man 24,8 g NaOH als ca. 50%ige Lösung in Wasser bei 5 - 10°C zulaufen, rührt 30 Minuten bei Raumtemperatur und dann 6 Stunden unter

Rückfluß nach. Man saugt ab und fällt das Filtrat in 5 Liter Methanol, wäscht mit Wasser nach und trocknet das Produkt bei 70°C unter Vakuum. Man erhält 130 bis 170 g halogeniertes s-Triazin als weißen Feststoff.

Beispiel 9

In einem Glaskolben werden 0,2 Mol Cyanurchlorid, 0,04 Mol Tetrabromhydrochinon und 0,52 Mol Tribromphenol in 400 ml Tetrahydrofuran vorgelegt. Dann läßt man 24,8 g Natronlauge, als ca. 50%ige Lösung in Wasser, bei +10°C zulaufen und rührt 18 Stunden bei Raumtemperatur nach. Danach destilliert man Tetrahydrofuran ab und nimmt den Rückstand in 500 ml 1,2-Dichloräthan auf. Man wäscht die organische Phase 3 mal mit je 100 ml Wasser, destilliert die Lösungsmittel ab. Es verbleiben 190 g Produktschmelze vom Bromgehalt 67,5 %.

Beispiele 10 bis 12 und Vergleichsversuche A bis D

Flammfestausrüsten von ABS (vgl. Tabelle 2) mit Mischungen von Verbindungen der Formel I und II.

Tabelle 1

| Beispiel | Molverhältnis Cyanurchlorid zu Tetrabromhydrochinon | (x)[1] Mol Tetrabromhydrochinon | (y)[1] Mol Tribromphenol | Endprodukt % Brom | Endprodukt Molgewicht[2] |
|---|---|---|---|---|---|
| 1 | 2,0 | 0,1 | 0,4 | 67,2 | 1980 |
| 2 | 2,22 | 0,09 | 0,42 | 67,0 | 1920 |
| 3 | 2,5 | 0,08 | 0,44 | 66,8 | 1780 |
| 4 | 3 | 0,066 | 0,466 | 66,9 | 1520 |
| 5 | 4 | 0,05 | 0,50 | 67,0 | 1510 |
| 6 | 5 | 0,04 | 0,52 | 67,1 | 1140 |
| 7 | 7 | 0,028 | 0,54 | 66,9 | 1250 |
| 8 | 8 | 0,025 | 0,55 | 66,8 | 1320 |

1) 2 (x) + (y) = 0,2 . 3

2) Mechrolab, Chloroform als Lösungsmittel, auf konz. =0 extrapoliert

Tabelle 2

| Beispiel | Flammschutzmittel aus Tabelle 1 [Gew.-Teile] | | Antimontrioxid [Gew.-Teile] | ABS [Gew.-Teile] | Schädigungsarbeit [Nm] | Oberflächenbelag nach 800 Stunden | Brandklasse |
|---|---|---|---|---|---|---|---|
| (erfindungsgemäß) | | | | | | | |
| 10 | 3 | 20,0 | 5 | 75 | 12 | nein | VE-0 |
| 11 | 4 | 20,0 | 5 | 75 | 16 | nein | VE-0 |
| 12 | 5 | 20,0 | 5 | 75 | 19 | nein | VE-0 |
| Vergleichsversuche (nicht erfindungsgemäß) | | | | | | | |
| A | 1 | 20,0 | 5 | 75 | 0,5 | nein | VE-0 |
| B | 2 | 20,0 | 5 | 75 | 7 | nein | VE-0 |
| C | 6 | 20,0 | 5 | 75 | 20 | ja | VE-0 |
| D | 7 | 20,0 | 5 | 75 | 20 | ja | VE-0 |

## Patentansprüche

1. Halogen enthaltende s-Triazinverbindungen der folgenden allgemeinen Formel I

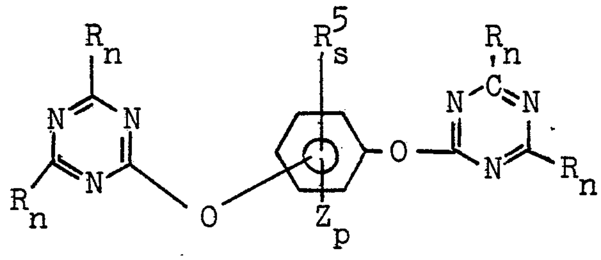

wobei die Reste und Indices folgende Bedeutung haben:

$R_n$: OH, $R^1$, $R^2$ oder $R^3$

$R^1$: 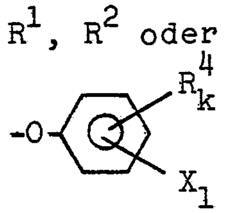

wobei gilt:

$R^4$: niedere Alkylgruppe oder niedere Halogenalkylgruppe

X: Brom oder Chlor

k, l: 0 oder ganze Zahlen von 1 bis 5 und ferner k+l max. 5;

$R^2$: 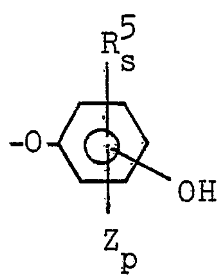

wobei gilt:

$R^5$: niedere Alkylgruppe oder niedere Halogenalkylgruppe

Z: Brom oder Chlor

s,p: 0 oder ganze Zahlen von 1 bis 4 und ferner s+p = max. 4

$R^3$: 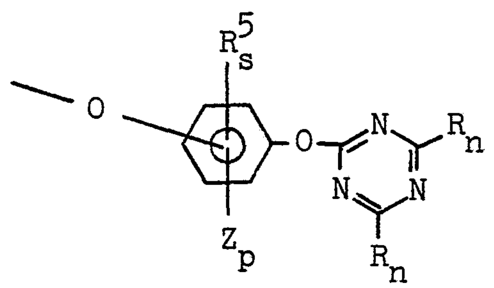

wobei $R_n$, $R^5$, Z, s und p die oben genannte Bedeutung haben und ferner gilt l+p = 1.

2. Verfahren zur Herstellung von Verbindungen gemäß Formel I von Patentanspruch 1 oder Mischungen von Verbindungen der allgemeinen Formel I und Halogen enthaltenden Tris(phenoxi)-s-triazinen der nachstehenden Formel II.

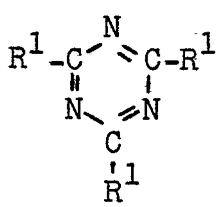

durch Umsetzen von

a) Cyanurchlorid

mit

b) einem Phenol der allgemeinen Formel III

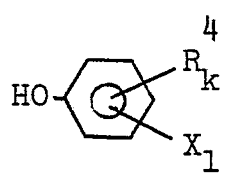

und

c) einem substituierten, zwei OH-Gruppen enthaltenden Benzolderivat der allgemeinen Formel IV

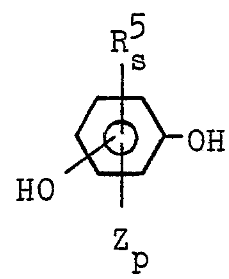

in Gegenwart von

d) mindestens 3 Äquivalenten einer Base, bezogen auf 1 Äquivalent Cyanurchlorid, gegebenenfalls in Gegenwart eines

e) organischen Lösungsmittels, das bevorzugt mit Wasser mischbar ist (Bedeutung der Reste und Indices wie Anspruch 1).

3. Verfahren nach Anspruch 2, dadurch gekennzeichnet, daß man Cyanurchlorid a) mit soviel Äquivalenten an b)+c), bezogen auf OH-Gruppen, umsetzt, daß auf 1 OH-Äquivalent 1 Cl-Äquivalent kommt.

4. Verfahren nach Anspruch 2, dadurch gekennzeichnet, daß man die Reaktanten a) und c) in einem Bereich des Molverhältnisses a/c von 1 bis 20 anwendet.

5. Verwendung von Halogen enthaltenden s-Triazinverbindungen der allgemeinen Formel I, gegebenenfalls in Mischung mit s-Triazinverbindungen der allgemeinen Formel II, für die Flammfestausrüstung von thermoplastischen Kunststoffen.

6. Verwendung von Halogen enthaltenden s-Triazinverbindungen der allgemeinen Formel I, gegebenenfalls in Mischung mit s-Triazinverbindungen der allgemeinen Formel II, zur Flammfestausrüstung von thermoplastischen Kunststoffen, zusammen mit einem üblichen Synergisten für das Flammschutzmittel in wirksamen Mengen.

7. Verwendung von Halogen enthaltenden s-Triazinverbindungen nach Anspruch 5 oder 6 für die Flammfestausrüstung von mit Kautschuk schlagfest modifizierten Styrol-Acrylnitril-Copolymerisaten (ABS-Harzen) mit der Maßgabe, daß als Flammschutzmittel eine Mischung der Verbindungen der allgemeinen Formel I und der Formel II in einem Anteil von 22 bis 53 Teilen von Verbindungen der Formel II, bezogen auf 100 Teile I+II, angewendet wird.

Europäisches Patentamt

**EUROPÄISCHER RECHERCHENBERICHT**

0050220

Nummer der Anmeldung

EP 81107474.9

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | FR - A - 1 566 675 (SOCIETE)<br>* Patentanspruch 1 *<br>-- | 1 |
| D | US - A - 3 843 650 (PEWS)<br>* Zusammenfassung *<br>-- | 1 |
| D | DE - A1 - 2 727 650 (ASAHI)<br>* Anspruch 1 *<br>-- | 1 |
| | US - A - 4 069 380 (EATON)<br>* Zusammenfassung *<br>& DE-A-2 641 953<br>-- | 1 |
| | US - A - 3 962 241 (HUNTER)<br>* Anspruch 1 *<br>& DE-A-2 434 790<br>-- | 1 |
| | DE - A - 2 155 453 (CIBA)<br>* Anspruch 1 *<br>-- | 1 |
| | DE - A - 1 770 387 (MINISTERUL)<br>* Anspruch 1 *<br>---- | 1 |

KLASSIFIKATION DER ANMELDUNG (Int. Cl.³)

C 07 D 251/34
C 08 K 5/34

RECHERCHIERTE SACHGEBIETE (Int. Cl.³)

C 07 D 251/00
C 08 K 5/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 05-02-1982 | HAMMER |